# EUROPEAN PATENT SPECIFICATION

(11) **EP 2 811 972 B1**
(45) Date of publication and mention of the grant of the patent: **03.02.2016**
(21) Application number: 13703795.8
(22) Date of filing: 07.02.2013
(51) Int. Cl.: A61K 8/44, A61Q 19/10, A61K 8/20

(54) **CALCIUM AND MAGNESIUM SALTS AS SQUEAKINESS ENHANCERS IN CLEANSING COMPOSITIONS**
CALCIUM- UND MAGNESIUMSALZEN ALS QUIETSCHVERSTÄRKER IN REINIGUNGSZUSAMMENSETZUNGEN
SELS DE CALCIUM OU DE MAGNÉSIUM COMME AMPLIFICATEURS DE CRISSEMENT DANS DES COMPOSITIONS NETTOYANTES

(30) Priority: 10.02.2012 US 201213370733
(43) Date of publication of application: 17.12.2014
(73) Proprietor: Unilever PLC, London, Greater London EC4Y 0DY (GB); Unilever N.V., 3013 AL Rotterdam (NL)
(72) Inventor: VETHAMUTHU, Martin, Swanson, Southbury, Connecticut 06488 (US); MUKHERJEE, Surajit, Trumbull, Connecticut 06611 (US); TIAN, Wei, Dong, West Roxbury, Massachusetts 02132 (US); DING, Junqi, Trumbull, Connecticut 06611 (US); PALLA-VENKATA, Chandra, Sekhar, Trumbull, Connecticut 06611 (US); DAVE, Rajendra, Mohanlal, Trumbull, Connecticut 06611 (US)
(74) Representative: Tansley, Sally Elizabeth
(86) International application number: PCT/EP2013/052469
(87) International publication number: WO 2013/117665

(56) References cited:
- EP-A1- 1 574 203
- EP-A1- 1 746 141
- WO-A1-97/01328
- WO-A1-2005/016304
- US-A1- 2007 066 501

## Description

### BACKGROUND OF THE INVENTION

### Field of the Invention

The present invention relates to skin and hair cleansing compositions in toilet bar, body wash, liquid hand cleanser, shampoo and related products.

### The Related Art

Soap has been a mainstay active for cleansers. It is cheap and efficient. But it can be harsh on the skin. Synthetic surfactants have been introduced as replacements for soap. Some of these generate lather equivalent to that of soap and have the further benefit of being milder.

Often the skin-feel properties of a synthetic surfactant are quite different than that of soap. Indeed, generally as mildness increases, the deposition of the synthetic surfactant and other moisturizers increases. This forms a protective barrier to the stripping of natural oils and fats from the epidermis.

A majority of consumers in Japan, and many in other countries, find the feel of synthetic detergents to be lacking. They consider moisturizing deposits as a sign of cleaning inefficiency. They need to be reassured of cleanliness by the traditional squeaky non-lubricated feel of soap.

US 2007/0066501 discloses a process for enhancing squeaky feel by reducing or eliminating surfactant micelles on dilution

### SUMMARY OF THE INVENTION

In accordance with the present invention, there is provided a foaming cleanser composition according to Claim 1.

A foaming cleanser composition is provided which includes:
(i) from 0.001 to 1% by weight of a calcium or magnesium salt;
(ii) from 0.1 to 15% by weight of an amphoteric surfactant; and
(iii) from 0.1 to 10% by weight of a C₈-C₂₂ acyl glycinate salt; and
   wherein the composition exhibits a UMT Test number of rubs to onset of stick-slip ranging from 1 to 12 under a 10 g load.

### DETAILED DESCRIPTION OF THE INVENTION

Now it has been found that amphoteric surfactants have a negative effect upon the stick-slip friction which generates a squeaky feel in a cleansing of the skin. We have found that small amounts of calcium or magnesium salts add squeakiness to the feel of amphoteric surfactant based cleansing systems while not detracting from mildness of the surfactants. Squeakiness has been correlated by us to the output from a Tribometer UMT friction/stick-slip tester. This instrument measures the change in number of rubs to onset of stick-slip between a base formula and a sample composition. Squeakiness is evidenced relative to the base formula by number of rubs to stick-slip ranging from 1 to 12, preferably from 1 to 9, optimally from 1 to 8, under a 10 g load.

A first component of the present compositions will be a calcium or magnesium salt to function as a squeakiness enhancing agent. Suitable, but not limiting, calcium or magnesium salts may be selected from the group consisting of calcium chloride, magnesium chloride, calcium carbonate, magnesium carbonate, calcium bicarbonate, magnesium bicarbonate, calcium sulphate, magnesium sulphate, calcium phosphate, magnesium phosphate, calcium hydroxide, magnesium hydroxide, calcium citrate, magnesium citrate, calcium tartrate, magnesium tartrate and combinations thereof. Inorganic salts are particularly preferred. Magnesium or calcium silicates may also be useful for many formulations, but for others the compositions may be negatively affected and in these the magnesium silicates should be absent. Amounts of the calcium or magnesium salt may range from 0.001 to 1%, preferably from 0.001 to 0.6%, more preferably from 0.01 to 0.3%, and optimally from 0.01 to 0.1 % by weight of the composition.

Another component of the compositions disclosed herein is that of an amphoteric surfactant. The term "amphoteric" is herein meant to also include what some refer to as zwitterionic type surfactants. Suitable amphoteric surfactants for use herein include, but are not limited to, derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one substituent contains an anionic group, e.g., carboxy, sulfonate, sulfate, phosphate, or phosphonate. Illustrative amphoterics are coco dimethyl carboxymethyl betaine, cocoamidopropyl betaine, cocobetaine, oleyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyethyl betaine, sodium cocoyl polyaminocarboxylate, sodium stearyl polyamidocarboxylate, sodium carboxymethyloleylpolypropylamine, N-disodium N-cocoyl-N-carboxymethoxyethyl-N-carboxymethylethylenediamine (INCI name: disodium cocoamphodiacetate), sodium cocoamphoacetate, and mixtures thereof. The sulfobetaines may include stearyl dimethyl sulfopropyl betaine, lauryl dimethyl sulfoethyl betaine, lauryl bis-(2-hydroxyethyl) sulfopropyl betaine and mixtures thereof. Most preferred is cocoamidopropyl betaine.

The amount of amphoteric surfactant present may range in amount from about 0.1 to about 15%, preferably from about 1 to about 10%, more preferably from about 2 to about 8%, and optimally from about 3 to about 6% by weight of the composition.

In accordance with the present invention, the composition also comprises a C₈-C₂₂ acyl glycinate salt. Cationic counterions to form the salt of the glycinate may be selected from sodium, potassium, ammonium, alkanolammonium and mixtures of these cations.

Suitable glycinate salts include sodium cocoylglycinate, potassium cocoylglycinate, sodium lauroylglycinate, potassium lauroylglycinate, sodium myristoylglycinate, potassium myristoylglycinate, sodium palmitoylglycinate, potassium palmitoylglycinate, sodium stearoylglycinate, potassium stearoylglycinate, ammonium cocoylglycinate and mixtures thereof. Amounts of the glycinate salt range from 0.1 to 10%, preferably from 1 to 6%, and optimally from 2 to 5% by weight of the composition.

Anionic and/or nonionic surfactants in addition to the amphoteric surfactant(s) may also be included in the compositions. Examples of anionic surfactants suitable for use herein include, but are not limited to, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, potassium lauryl sulfate, sodium trideceth sulfate, sodium methyl lauroyl taurate, sodium lauroyl isethionate, sodium laureth sulfosuccinate, sodium lauroyl sulfosuccinate, sodium tridecyl benzene sulfonate, sodium dodecyl benzene sulfonate, and mixtures thereof.

The anionic surfactant may be, for example, an aliphatic sulfonate, such as a primary C₈-C₂₂ alkane sulfonate, primary C₈-C₂₂ alkane disulfonate, C₈-C₂₂ alkene sulfonate, C₈-C₂₂ hydroxyalkane sulfonate or alkyl glyceryl ether sulfonate. Amounts of anionic surfactant range from 0.1 to 15%, preferably from 1 to 10%, and more preferably from 3 to 8% by weight of the composition.

Nonionic surfactants which may be used include the reaction products of compounds having a hydrophobic group and a reactive hydrogen atom. Exemplative are alcohols, acids, amides or alkyl phenols reacted with alkylene oxides, especially ethylene oxide either alone or with propylene oxide. Specific nonionics are C₆-C₂₂ alkyl phenols-ethylene oxide condensates, the condensation products of C₈-C₁₈ aliphatic primary or secondary linear or branched alcohols with ethylene oxide, and products made by condensation of ethylene oxide with the reaction products of propylene oxide and ethylenediamine. Other nonionics include long chain tertiary amine oxides, long chain tertiary phosphine oxides and dialkyl sulphoxides. Also useful are the alkyl polysaccharides.

Surfactants should be chosen which will allow the compositions to have a SITA Foam Test Value ranging from 200 to 800, preferably from 300 to 700, and optimally between 400 and 600 ml. SITA Foam Tester R-2000 Model from Future Digital Scientific Corp. is normally utilized for this evaluation. The experiment protocol includes loading 10 g of product (no pre-dilution) into a measuring cylinder. Thereinto is added 250 ml water at 40-45° C. The foam volume is that recorded after stirring the sample for 30 seconds at 1000 rpm using a rotor. Final foam volume is obtained as the average of ten repeats of measuring the foam volume upon stirring the sample for the 30 seconds at 1000 rpm.

C₈-C₂₂ fatty acids may also be included in compositions of this invention. Suitable fatty acids are lauric acid, myristic acid, palmitic, stearic, oleic, linoleic, behenic and acid combinations thereof. Particularly useful are the C₁₂-C₁₄ fatty acids such as lauric acid and myristic acid. Amounts of the fatty acid may range from about 0.1 to about 15%, preferably from about 0.5 to about 10%, and optimally from about 1 to about 5% by weight of the composition.

Water may be present in the compositions in amounts from about 5 to about 95%, preferably from about 50 to about 90%, and optimally from 65 to 85% by weight.

Water soluble/dispersible polymers are an optional ingredient that may be included in the compositions of the invention. These polymers can be cationic, anionic, amphoteric or nonionic types with molecular weights higher than 100,000 Dalton. They are known to increase the viscosity and stability of liquid cleanser compositions, to enhance in-use and after-use skin sensory feels, and to enhance lather creaminess and lather stability. Amount of the polymers when present may range from 0.1 to 10% by weight of the composition.

Examples of water soluble/ or dispersible polymers include the carbohydrate gums such as cellulose gum, microcrystalline cellulose, cellulose gel, hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, xanthan gum and mixtures thereof; modified and nonmodified starch granules and pregelatinized cold water soluble starch; emulsion polymers such as Aculyn® 28, Aculyn® 22 or Carbopol ®Aqua SF1; cationic polymer such as modified polysaccharides including cationic guar available from Rhone Poulenc under the trade name Jaguar C13S, Jaguar C14S, Jaguar C17, or Jaguar C16; cationic modified cellulose such as UCARE Polymer JR 30 or JR 40 from Amerchol; N-Hance® 3000, N-Hance® 3196, N-Hance® GPX 215 or N-Hance® GPX 196 from Hercules; synthetic cationic polymer such as Merquat® 100, Merquat® 280, Merquat® 281 and Merquat® 550 sold by Nalco; cationic starches such as StaLok® 100, 200, 300 and 400 sold by Staley Inc.; cationic galactomannans such as Galactasol® 800 series by Henkel, Inc.; Quadrosoft® LM-200; and Polyquatemium-24. Also suitable are high molecular weight polyethylene glycols such as Polyox® WSR-205 (PEG 14M), Polyox® WSR-N-60K (PEG 45), and Polyox® WSR-301 (PEG 90M).

Water-soluble skin benefit agents may optionally be formulated into the compositions of the invention. A variety of water-soluble skin benefit agents can be used and the level can be from 0.1 to 50% but preferably from 1 to 30% by weight of the composition. These materials include, but are not limited to, polyhydroxy alcohols. Preferred water soluble skin benefit agents are glycerin, sorbitol and polyethylene glycol.

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflowerseed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatabilities between the preservatives and other ingredients. Preservatives are preferably employed in amounts ranging from 0.01 % to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

It should be noted that in specifying any range of concentration or amount, any particular upper concentration can be associated with any particular lower concentration or amount.

The following examples will more fully illustrate the embodiments of this invention. All parts, percentages and proportions referred to herein and in the appended claims are by weight unless otherwise illustrated.

### EXAMPLE 1

Evaluations of squeakiness were performed by use of a Tribometer UMT device. The procedure is described below.

### Tribometer UMT Procedure

The Tribometer UMT is a device measuring loading force and friction force between upper and lower test specimens with a set of two dimensional force sensors. The equipment is available from the Center for Tribology, Inc., Campbell, CA 95008. A slider is utilized to move the upper specimen against the stationary lower specimen. The upper specimen is connected to a vertical linear motion system that has a travel length of 150 mm. Ultra-accurate strain gauge sensors perform simultaneous measurements of load and torque in two axes. Resolution is 0.00003% of the full scale and achieves very high repeatability. A normal load sensor provides feedback to the vertical motion controller, actively adjusting sample position to ensure a constant load during testing.

For the present experiments, a FL 50g sensor was employed with a 10 g load. The sensor probe was fitted with a glass lens which was covered by a solid grip liner. Speed was set at 10 mm/s with a travel distance of 50 mm.

A synthetic substrate known as VitroSkinT"' strip 3 cm x 11 cm was wetted in water (about 45°C) for 2 minutes and clamped onto a rubber plate. A sample (2 mg/cm²) was placed on the surface of the VitroSkin™ and rubbed under water 10 times to achieve even distribution. Water is then added to the evaluation container and the probe head soaked under water. The probe was programmed to contact the VitroSkin™ with 20 g load and for rubbing onto the surface in one direction with 10 mm/s speed, raised up 10 mm and moved back to an initial position.

The probe is then rubbed on the VitroSkin™ for 40 times under water. Rubbing is stopped when friction exceeds the limitation weight (45 g), or after the stick-slip moves to a large intensity. The base line is a single application step before water wash.

The friction and loading force are recorded. The mean friction force and the stick-slip amplitude is calculated.

### Samples

A set of formulas were evaluated with the Tribomoter UMT equipment. Formulations of these examples are recorded in Table I.

**TABLE I**

| | Sample (Weight %) | | | | |
|---|---|---|---|---|---|
| Components | A | B | C | D | E |
| Potassium Cocoylglycinate | 5 | -- | 5 | -- | 5 |
| Sodium Cocoylglycinate | -- | 5 | -- | 5 | -- |
| Cocoamidopropyl Betaine | -- | -- | 4.8 | 4.8 | 4.8 |
| Lauric Acid | -- | -- | -- | 1 | 1 |
| Sodium Sarcosinate | -- | -- | -- | -- | 1 |
| Aqua SF-1® Thickener | -- | -- | -- | -- | 1.5 |
| Water | Balance | Balance | Balance | Balance | Balance |

Samples A through E were evaluated on the Tribometer UMT instrument at 10 g load. Results are recorded in Table II.

**TABLE II**

| | No Calcium Salt | Calcium Salt Present |
|---|---|---|
| Sample No. | No. of Rubs To Onset of Stick-Slip | No. of Rubs To Onset of Stick-Slip |
| A | >20 | 1 |
| B | 1 | 1 |
| C | 13 | 6 |
| D | 11 | 8 |
| E | 15 | 2 |

The effect of 0.01% calcium chloride was most pronounced against Sample A having potassium cocoyl glycinate as surfactant. The sodium cocoyl glycinate itself (Sample B) exhibited good stick-slip characteristics. Addition of the cocoamidopropyl betaine (Sample C) degraded stick-slip properties. Here the addition of 0.01 % calcium chloride significantly improved the friction properties. Similarly, positive effects of calcium salt were noted in Samples D and E.

### EXAMPLE 2

Effects of concentration and salt types were evaluated in a control base AGB whose composition is outlined in Table III. The amount of glycerin was adjusted downward dependant on the amount of added calcium salt in a test sample.

**TABLE III**

| Control Base AGB | |
|---|---|
| Component | Control Base AGB (Weight %) |
| Cocoamidopropyl Betaine | 4.8 |
| Sodium Cocoyl Glycinate | 4.0 |
| Lauric Acid | 2.4 |
| Glycerin* | 2.0 |
| Fragrance | 1.0 |
| Polyacrylic Thickener | 1.5 |
| Water | qs |

Evaluations were conducted with a Tribometer UMT instrument utilizing a 10 g load. Results are reported in a Table IV.

**TABLE IV**

| Salt | Concentration (percentage) | No. rubs for stick-slip |
|---|---|---|
| AGB base - No salt | 0 | 9 |
| Calcium Chloride | 0.005 | 12 |
| | 0.01 | 3 |
| | 0.05 | 5 |
| | 0.1 | 10 |
| | 0.2 | 15 |
| | 0.3 | >20 |
| | 0.6 | >20 |
| | 1 | >20 |
| | | |
| Magnesium Carbonate | 0.01 | 12 |
| | 0.1 | 7 |
| | 0.3 | 10 |
| | 0.6 | 7 |
| | 1 | 12 |
| | 3 | >20 |
| | | |
| Calcium Citrate | 0.01 | 12 |
| | 0.1 | 10 |
| | 0.3 | 14 |
| | 0.6 | 11 |
| | 1 | 9 |
| | | |
| Calcium Tartrate | 0.01 | 9 |
| | 0.1 | 11 |
| | 0.3 | 7 |
| | 0.6 | 6 |
| | 1 | 7 |

Evident from Table IV is that only small amounts of calcium salts are necessary to impart good friction properties. Calcium chloride was better than the calcium citrate and tartrate salts. Magnesium carbonate as a salt was also found useful.

## Claims

1. A foaming cleanser composition comprising:
(i) from 0.001 to 1% by weight of a calcium or magnesium salt;
(ii) from 0.1 to 15% by weight of an amphoteric surfactant;
(iii) from 0.1 to 10% by weight of a C₈-C₂₂ acyl glycinate salt; and
wherein the composition exhibits a UMT Test number of rubs to onset of stick-slip ranging from 1 to 12 under a 10 g load.

2. The composition according to claim 1 wherein the calcium or magnesium salt is selected from the group consisting of calcium chloride, magnesium chloride, calcium carbonate, magnesium carbonate, calcium bicarbonate, magnesium bicarbonate, calcium sulphate, magnesium sulphate, calcium phosphate, magnesium phosphate, calcium hydroxide, magnesium hydroxide, calcium citrate, magnesium citrate, calcium tartrate, magnesium tartrate and combinations thereof.

3. The composition according to claim 1 wherein the calcium or magnesium salt is present in an amount from 0.01 to 0.3% by weight of the composition.

4. The composition according to claim 1 wherein the amphoteric surfactant is a betaine.

5. The composition according to claim 4 wherein the betaine surfactant is cocoamidopropyl betaine.

6. The composition according to claim 1 wherein the amphoteric surfactant is present in an amount from 1 to 10% by weight of the composition.

7. The composition according to claim 1 further comprising a C₈-C₂₂ fatty acid.

8. The composition according to claim 7 wherein the fatty acid ranges in amount from 0.1 to 15% by weight of the composition.

9. The composition according to claim 1 wherein the UMT Test exhibits a number of rubs to onset of stick-slip ranging from 1 to 9.

## Patentansprüche

1. Schaumreinigerzusammensetzung, umfassend
(i) von 0,001 bis 1 Gewichts-% eines Calcium- oder Magnesiumsalzes,
(ii) von 0,1 bis 15 Gewichts-% eines amphoteren Tensids,
(iii) von 0,1 bis 10 Gewichts-% eines C₈-C₂₂-Acylglycinatsalzes und
wobei die UMT-Test-Zahl der Reibungen bis zum Einsetzen des Ruckgleitens unter einer 10 g-Last zwischen 1 und 12 liegt.

2. Zusammensetzung nach Anspruch 1, wobei das Calcium- oder Magnesiumsalz aus der Gruppe ausgewählt ist, bestehend aus Calciumchlorid, Magnesiumchlorid, Calciumcarbonat, Magnesiumcarbonat, Calciumbicarbonat, Magnesiumbicarbonat, Calciumsulfat, Magnesiumsulfat, Calciumphosphat, Magnesiumphosphat, Calciumhydroxid, Magnesiumhydroxid, Calciumcitrat, Magnesiumcitrat, Calciumtartrat, Magnesiumtartrat und Kombinationen davon.

3. Zusammensetzung nach Anspruch 1, worin das Calcium- oder Magnesiumsalz in einer Menge von 0,01 bis 0,3 Gewichts-% der Zusammensetzung vorliegt.

4. Zusammensetzung nach Anspruch 1, worin das amphotere Tensid ein Betain ist.

5. Zusammensetzung nach Anspruch 4, worin das Betain-Tensid Cocoamidopropylbetain ist.

6. Zusammensetzung nach Anspruch 1, worin das amphotere Tensid in einer Menge von 1 bis 10 Gewichts-% der Zusammensetzung vorliegt.

7. Zusammensetzung nach Anspruch 1, die des Weiteren eine C₈-C₂₂-Fettsäure umfasst.

8. Zusammensetzung nach Anspruch 7, worin die Menge der Fettsäure zwischen 0,1 und 15 Gew.-% der Zusammensetzung liegt.

9. Zusammensetzung nach Anspruch 1, worin der UMT-Test eine Zahl von Reibungen bis zum Einsetzen des Ruckgleitens zwischen 1 bis 9 zeigt.

## Revendications

1. Composition de nettoyant moussant comprenant :
(i) de 0,001 à 1 % en masse d'un sel de calcium ou de magnésium ;
(ii) de 0,1 à 15 % en masse d'un tensioactif amphotère ;
(iii) de 0,1 à 10 % en masse d'un sel de glycinate d'acyle en C₈-C₂₂ ; et
dans laquelle la composition présente un nombre de Test UMT de frottements jusqu'au début de collé-glissé compris entre 1 à 12 sous une charge de 10 g.

2. Composition selon la revendication 1, dans laquelle le sel de calcium ou de magnésium est choisi dans le groupe constitué de chlorure de calcium, chlorure de magnésium, carbonate de calcium, carbonate de magnésium, bicarbonate de calcium, bicarbonate de magnésium, sulfate de calcium, sulfate de magnésium, phosphate de calcium, phosphate de magnésium, hydroxyde de calcium, hydroxyde de magnésium, citrate de calcium, citrate de magnésium, tartrate de calcium, tartrate de magnésium et combinaisons de ceux-ci.

3. Composition selon la revendication 1, dans laquelle le sel de calcium ou de magnésium est présent dans une quantité de 0,01 à 0,3 % en masse de la composition.

4. Composition selon la revendication 1, dans laquelle le tensioactif amphotère est une bétaïne.

5. Composition selon la revendication 4, dans laquelle le tensioactif de bétaïne est la cocoamidopropylbétaïne.

6. Composition selon la revendication 1, dans laquelle le tensioactif amphotère est présent dans une quantité de 1 à 10 % en masse de la composition.

7. Composition selon la revendication 1 comprenant de plus un acide gras en C₈-C₂₂.

8. Composition selon la revendication 7, dans laquelle l'acide gras est compris dans une quantité de 0,1 à 15 % en masse de la composition.

9. Composition selon la revendication 1, dans laquelle le Test UMT présente un nombre de frottements jusqu'au début de collé-glissé compris entre 1 à 9.
